# EUROPÄISCHE PATENTSCHRIFT

(11) **EP 2 024 418 B1**
(45) Veröffentlichungstag und Bekanntmachung des Hinweises auf die Patenterteilung: **17.11.2010**
(21) Anmeldenummer: 07728781.1
(22) Anmeldetag: 04.05.2007
(51) Int. Cl.: C08G 69/44

(54) **MULTIBLOCKCOPOLYMERE MIT FORMGEDÄCHTNISEIGENSCHAFTEN**
MULTIBLOCK COPOLYMERS WITH SHAPE-MEMORY PROPERTIES
COPOLYMÈRES MULTIBLOCS AYANT DES PROPRIÉTÉS DE MÉMOIRE DE FORME

(30) Priorität: 15.05.2006 DE 102006023365
(43) Veröffentlichungstag der Anmeldung: 18.02.2009
(73) Patentinhaber: GKSS-Forschungszentrum Geesthacht GmbH, 21502 Geesthacht (DE)
(72) Erfinder: FENG, Yakai School of Chemical Engineering and Technology, Tianjin, 30072 (CN); KELCH, Steffen, CH-8102 Oberengstringen (CH); LENDLEIN, Andreas, 14167 Berlin (DE)
(74) Vertreter: Neigenfink, Jan Cornelius
(86) Internationale Anmeldenummer: PCT/EP2007/054328
(87) Internationale Veröffentlichungsnummer: WO 2007/131893

(56) Entgegenhaltungen:
- EP-A1- 1 642 921
- WO-A-2004/090042
- WO-A-2005/059003
- US-B1- 6 388 043
- SHIRAHAMA H UMEMOTO K YASUDA H: "Synthesis and enzymatic degradation of optically active depsipeptide copolymers" JOURNAL OF BIOMATERIALS SCIENCE. POLYMER EDITION, VSP, UTRECHT, NL, Bd. 10, Nr. 6, 1999, Seiten 621-639, XP002956695 ISSN: 0920-5063

## Beschreibung

Die Erfindung betrifft ein Multiblockcopolymer mit Formgedächtniseigenschaften sowie eine synthetische Vorstufe des Multiblockcopolymers.

### Hintergrund der Erfindung und Stand der Technik

Formgedächtnismaterialien sind Werkstoffe, die unter Einwirkung eines externen Stimulus ihre äußere Form ändern können. Für die vorliegende Erfindung von Bedeutung sind thermosensitive Formgedächtniskunststoffe, die auch als Shape-Memory-Polymere bezeichnet werden. Der Formgedächtniseffekt ist keine spezifische Stoffeigenschaft einzelner Polymere; vielmehr resultiert er unmittelbar aus der Kombination von Polymerstruktur und -morphologie und einer Verarbeitungs- und Programmierungstechnik.

Bei Elastomeren erhält man eine Formgedächtnisfunktionalität, wenn es gelingt, das Elastomer in seiner deformierten Gestalt in einem bestimmten Temperaturbereich zu stabilisieren. Dies kann erreicht werden, indem man zum Beispiel Netzketten als molekulare Schalter benutzt. Eine Möglichkeit für eine Schalterfunktion ist ein thermischer Übergang (T_{Trans}) in der Netzkette in dem für die Anwendung interessanten Temperaturbereich. Ist die Temperatur höher als T_{Trans} des Schaltsegments, sind die Segmente flexibel und das Polymer kann elastisch deformiert werden. Die temporäre Form wird durch Abkühlen unter T_{Trans} fixiert. Wird das Polymer wieder erwärmt, wird die permanente Form wieder hergestellt.

Ein bedeutsames Anwendungsgebiet für Formgedächtnispolymere ist der biomedizinische Bereich. Synthetische, abbaubare Implantatmaterialien haben in den letzen 30 Jahren zu entscheidenden Fortschritten bei den verschiedensten Therapien geführt. Abbaubare Implantatmaterialien umfassen beispielsweise Polyhydroxycarbonsäuren, wie Polyglycolid oder die Copolyester von L-Milchsäure und Glycolsäure. Abbaubare Formgedächtnispolymere könnten beim Einsatz als abbaubare Implantatmaterialien deren Attraktivität weiter erhöhen; sie bieten ein hohes Anwendungspotential in der minimalinvasiven Medizin. Abbaubare Implantate könnten beispielsweise in komprimierter (temporärer) Form durch eine kleine Inzision in den Körper eingebracht werden, wo sie ihre gespeicherte, anwendungsrelevante Gestalt nach Erwärmung auf Körpertemperatur annehmen. Nach einer vorgegebenen Zeit wird das Implantat abgebaut; eine zweite Operation zu seiner Entfernung entfällt.

Gerade mit Hinblick auf eine derartige Anwendung ist das Risiko einer toxischen Wirkung des Formgedächtnismaterials und seiner Abbauprodukte bedeutsam; diese sollten biokompatibel sein.

In diesem Zusammenhang werden Poly(ε-caprolacton)diole mit Schmelztemperaturen zwischen 46 und 64°C sowie amorphe Copolyester von Diglycoliden mit Glasübergangstemperaturen im Bereich von 35 bis 50°C als geeignete Schaltsegmente für abbaubare Formgedächtnispolymere beschrieben. Die genannten Schaltsegmente weisen ein mittleres Molekulargewicht M_{w} zwischen 500 und 10.000 auf und verfügen über einen für biomedizinische Anwendungen günstigen thermischen Übergang der Schaltsegmente im Bereich zwischen Raumtemperatur und Körpertemperatur.

Ein biokompatibles und zugleich bioabbaubares Multiblockcopolymer mit Formgedächtniseigenschaften kann aus kristallisierbaren Hartsegmenten aus Poly(p-dioxanon) und einem amorphen Schaltsegment, wie einem kristallisierbaren Poly(ε-caprolacton)-Segment erhalten werden. Die thermoplastischen Elastomere werden mittels Cokondensation von zwei verschiedenen Makrodiolen mit einer difunktionellen Verknüpfungseinheit (zum Beispiel Diisocyanat, Disäuredichlorid oder Phosgen) hergestellt. Um die gewünschten mechanischen Eigenschaften zu erhalten, ist es von entscheidender Bedeutung, hohe Molekulargewichte M_{w} im Bereich von 100.000 g/mol zu erreichen. Molekulare Parameter dieses Polymersystems sind Molekulargewicht, Mikrostruktur (Sequenz) und Comonomerverhältnis der Makrodiole sowie der Hartsegmentanteil im Multiblockcopolymer.

Trotz der geschilderten Fortschritte auf dem Gebiet besteht nach wie vor ein erheblicher Bedarf an Formgedächtnispolymeren, die im Körper hydrolytisch abbaubar sind, deren Abbauprodukte toxikologisch unbedenklich sind und die weiterhin günstige Eigenschaften für den geplanten Einsatzzweck aufweisen, wie beispielsweise Schalttemperaturen im Bereich von 30 bis 60°C und Verarbeitungstemperaturen von bis zu 200°C bei biomedizinischen Applikationen.

Aufgabe der vorliegenden Erfindung ist es daher, neue biodegradierbare Formgedächtnismaterialien bereitzustellen, die verbesserte oder zumindest gleichwertige Eigenschaften im Vergleich zu den bekannten Materialien aufweisen.

### Erfindungsgemäße Lösung

Nach einem ersten Aspekt der Erfindung wird diese Aufgabe durch das Multiblockcopolymer mit Formgedächtniseigenschaften nach Anspruch 1 gelöst. Das erfindungsgemäße Multiblockcopolymer enthält
(i) ein Poly(depsipeptid)-Segment mit einem mittleren Molekulargewicht M_{w} im Bereich von 1.000 bis 20.000 g/mol; sowie
(ii) ein Poly(ε-caprolacton)-Segment mit einem mittleren Molekulargewicht M_{w} im Bereich von 1.000 bis 10.000 g/mol.

Das erfindungsgemäße lineare Multiblockcopolymer zeichnet sich durch die Gegenwart eines Poly(depsipeptid)-Segments aus, das hydrolytisch abbaubar ist, wobei die Abbauprodukte, nämlich Aminosäuren und Hydroxycarbonsäuren, von guter biologischer Verträglichkeit sein dürften. Die beim hydrolytischen Abbau entstehenden Aminosäuren sind in der Lage, als Säure/Base-Puffer zu wirken, und so die Acidität der beim hydrolytischen Abbau entstehenden Hydroxycarbonsäuren abzupuffern. Dieser Mechanismus könnte eine Möglichkeit darstellen, den Verlauf der Wundheilung günstig zu beeinflussen, da die Freisetzung von sauren Abbauprodukten die auftretenden Entzündungsprozesse im allgemeinen verstärkt. Auch die Ausbildung kationischer Oberflächenladungen in Polymeren mit Poly(depsipetid)blöcken während des hydrolytischen Abbaus könnte gezielt eingesetzt werden, um den Wundheilungsprozess zu moderieren. Das Poly(depsipeptid)-Segment kann als Hart- und/oder Schaltsegment in dem Multiblockcopolymer agieren. Beim Einsatz als Schaltsegment wird die Glasübergangstemperatur (üblicherweise im Temperaturbereich zwischen 40 und 60°C) des amorphen Anteils der von dem Poly(depsipeptid)-Segmenten bestimmten Phase als Schalttemperatur genutzt. Die Kombination von Poly(depsipeptid)-Segmenten und Poly(ε-caprolacton)-Segmenten in Multiblockcopolymeren liefert ein hydrolytisch abbaubares thermoplastisches Elastomer mit Formgedächtniseigenschaften und Schalttemperaturen im Bereich von 30 bis 90 °C sowie Verarbeitungstemperaturen bis zu 200 °C im Fall von Hartsegmenten bildenden Blöcken auf Basis von Leucin und Diglycolid. Sowohl Hart- als auch Schaltsegmente sind hydrolytisch abbaubar gestaltet.

Die angegeben Molekulargewichte sind mittels Gelpermeationschromatographie (GPC) zu bestimmen. Die Bestimmung kann ergänzend anhand des ¹H NMR-Spektrums erfolgen.

Bevorzugt ist (i) ein Poly(depsipeptid)-Segment der Formel (1): 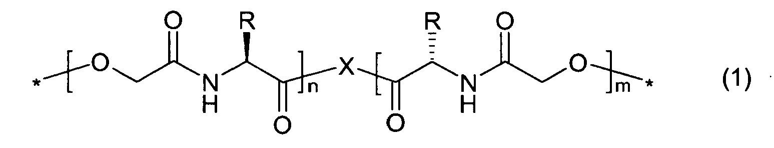 wobei X eine Brücke ausgewählt aus der Gruppe: 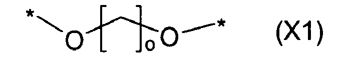 und 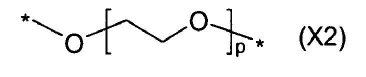 ist, mit o = 2 - 20 und p = 1 - 10;
R für eine Gruppe ausgewählt aus H oder einem verzeigten oder unverzweigten C₁-C₁₀-Alkylrest steht; und
n und m so vorgegeben sind, dass das Poly(depsipeptid)-Segment ein mittleres Molekulargewicht M_{w} im Bereich von 1.000 bis 20.000 g/mol aufweist.
Ferner steht X vorzugsweise für 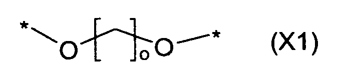 mit o = 8; oder für 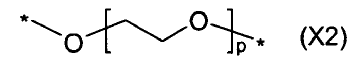 mit p = 1. Poly(depsipeptid)-Segmente mit den genannten Varianten des zentralen Brückenelements (Starters) lassen sich synthetisch leicht darstellen und weisen ersten Untersuchungen zu Folge für die Anwendung im medizin-technischen Gebiet günstige Materialeigenschaften auf.

Bevorzugt ist ferner, wenn R in Formel (1) für H, Methyl, 1-Methylethyl, 2-Methylpropyl oder 1-Methylpropyl steht. Hierdurch kann zum einen bei der Herstellung der Poly(depsipeptid)-Segmente auf an sich bekannte Synthesevorschriften zur ringöffnenden Polymerisation von Morpholin-2,5-dion-Derivaten mit einem entsprechenden die Brücke bildenden Diol als Starter zurückgegriffen werden. Zum anderen entsprechen die erzeugten Monomereinheiten im Poly(depsipeptid)-Segment den natürlichen Aminosäuren Glycin, Alanin, Valin, Leucin und Isoleucin, so dass eine hohe Biokompatibilität des Polymers und seiner Abbauprodukte zu erwarten ist.

Bevorzugt entspricht ferner - insbesondere auch in Kombination mit jeder der vorgenannten Variationen im Poly(depsipeptid)-Segment - das Poly(ε-caprolacton)-Segment der Formel (2) 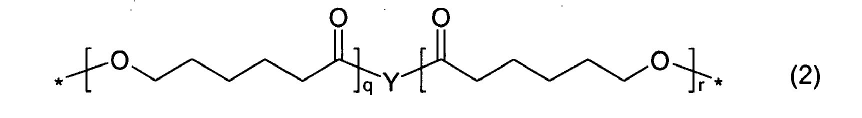 ist, wobei Y für 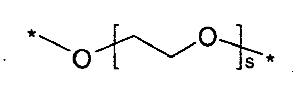 steht, mit s = 1 - 10; und
q und r so vorgegeben sind, dass das Poly(ε-caprolacton)-Segment ein mittleres Molekulargewicht M_{w} im Bereich von 1.000 bis 10.000 g/mol aufweist. Vorzugsweise ist s = 2.

Vorzugsweise sind die Poly(depsipeptid)-Segmente und Poly(ε-caprolacton)-Segmente im Multiblockcopolymer über Brücken der Formeln (3a) und/oder (3b) 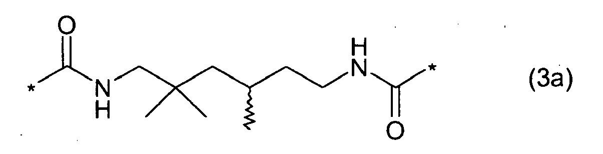 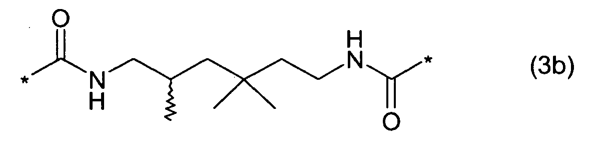 gekuppelt.

Ferner ist bevorzugt, wenn ein Gewichtsverhältnis der Poly(depsipeptid)-Segmente zu den Poly(ε-caprolacton)-Segmenten im Bereich von 1:1 bis 1:10 liegt.

Schließlich ist bevorzugt, wenn ein mittleres Molekulargewicht M_{w} des Multiblockcopolymers im Bereich von 10.000 bis 100.000 g/mol liegt.

Ein zweiter Aspekt der Erfindung richtet sich auf das als Zwischenprodukt der Synthese anfallende Poly(depsipeptid) der Formel (4): 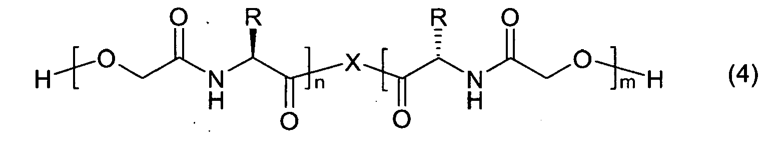 wobei X eine Brücke ausgewählt aus der Gruppe: 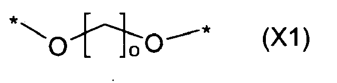 und 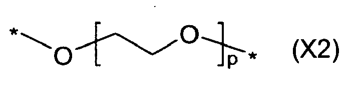 ist, mit o = 2 - 20 und p = 1 - 10;
R für eine Gruppe ausgewählt aus H oder einem verzeigten oder unverzweigten C₁-C₁₀-Alkylrest steht; und
n und m so vorgegeben sind, dass das Poly(depsipeptid) ein mittleres Molekulargewicht M_{w} im Bereich von 1.000 bis 20.000 g/mol aufweist. Hinsichtlich bevorzugter Varianten der erfindungsgemäßen Poly(depsipeptide) wird auf die obig bereits bei der Beschreibung des Muliblockpolymers beschriebenen bevorzugten Ausführungsformen der Brücken X1 und X2 sowie des Restes R verwiesen.

Ein dritter Aspekt der Erfindung liegt in der Verwendung des erfindungsgemäßen Multiblockcopolymers der obig beschriebenen Art als Implantatwerkstoff, als Polymermatrix für die kontrollierte Wirkstofffreisetzung (Wirkstoffdepots und Coatings zur Wirkstoffverkapselung) und als Material zur Herstellung von Gerüst- und Leitstrukturen (Polymer Scaffolds und alloplastische Leitschienen) für das Tissue Engineering.

Die Erfindung wird nachfolgend anhand von Ausführungsbeispielen und dazugehörigen Zeichnungen näher erläutert, Es zeigen:
- Figur 1: AFM-Abbildungen einer Oberfläche aus einem PCL/PIBMD- Multiblockcopolymer bei verschiedenen Temperaturen;
- Figur 2: eine Serie von Fotographien zur Illustration des makroskopischen Gedächtniseffekts beim Multiblockcopolymer (PCL/PIBMD); und
- Figur 3: einen zyklischen, thermomechanischen Zug-Dehnungs-Tests des Multiblock- copolymers (PCL/PIBMD).

### Synthese von Poly(depsipeptiden)

Poly(depsipeptide) sind alternierende Copolymere von α-Aminosäuren und α-Hydroxysäuren. Verschiedene Kombinationen von α-Aminosäuren (zum Beispiel L-Leucin, L-Valin, Glycin, L-Lysin oder L-Glutaminsäure) und einer α-Hydroxycarbonsäure (Glycolsäure, L,L-Dilactid oder rac-Dilactid) lassen sich zu neuen Werkstoffen nicht-toxischer und biodegradierbarer Natur umsetzen. Ein bekannter synthetischer Zugang zu Poly(depsipeptiden) ist die ringöffnende Polymerisation von Morpholin-2,5-dion-Derivaten in Gegenwart von Zinndioctanoat (Sn(oct)₂) als Katalysator. Weiterhin wurde über eine enzymatisch katalysierte ringöffnende Polymerisation von Morpholin-2,5-dionen berichtet. Bekannt sind ferner Blockcopolymere von 3-(S)-Isopropyl-morpholin-2,5-dion und Polyethylenoxid (PEO), die durch ringöffnende Polymerisation zugänglich sind. Poly(α-hydroxyalkonate), wie Poly(L-lactide) oder Copolymere von L,L-Dilactiden und Diglycoliden, werden als resorbierbare Implantatwerkstoffe, biodegradierbares Nahtmaterial und Matrix für eine kontrollierte Wirkstofffreigabe eingesetzt. Thermoplastische Multiblockcopolymere mit semikristalline Phasen bildenden Poly(ε-caprolacton)- und Poly(p-dioxanon)-Blöcken sowie AB-Polymernetzwerke auf Basis von semikristallinen Poly(ε-caprolacton)-Kettensegmenten sind als biodegradierbare Gedächtnispolymere beschrieben worden (A. Lendlein et al., Proc. Natl. Acad. Sci. USA, 2001, 98(3), 842; A. Lendlein et al., Science 2002, 296(5573), 1673). Biodegradierbare amorphe Poly[(rac-lactid)-ran-glycolid]-urethan-Netzwerke mit Formgedächtniseigenschaften wurden durch Kupplung mit sternförmigen Oligomeren unter Verwendung eines isomeren Gemisches von 2,2,4- und 2,4,4-Trimethylhexamethylendiisocyanat (TMDI) synthetisiert (A. Lendlein et al. Angew. Chem. 2005, 117, 1212).

### Synthese von Poly(3S-isobutyl-morpholin-2,5-dion)diol (PIBMD)

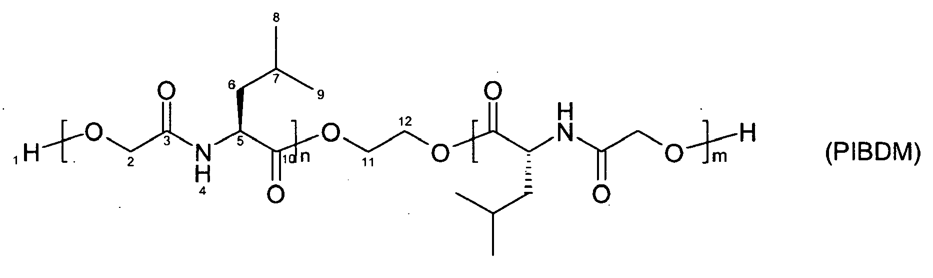

Die Polymerisation wurde in einem trockenen Glaskolben mit einem Rührstab durchgeführt. Der Kolben wurde auf 50°C, erhitzt, evakuiert und mit trocknem Stickstoff gespült. Der Kolben wurde mit 31,3 g-3S-Isobutyl-morpholin-2,5-dion (IBMD), 0,349 mL Ethylenglykol und 4 ml einer 0,3-molaren Sn(oct)₂-Lösung beschickt. Der Kolben wurde dann evakuiert und mehrfach mit trockenem Stickstoff gespült. Das Reaktionsgemisch wurde unter Stickstoff belassen und mittels eines Ölbades auf 140°C erhitzt. Nach 24 Stunden wurde der Kolben aus dem Ölbad entfernt und auf Raumtemperatur abgekühlt. Das Produkt wurde in 100 mL DMF gelöst und in 1 L Diethylether ausgefällt. Das erhaltene Polymer wurde gesammelt und unter Vakuum bei Raumtemperatur über 24 Stunden getrocknet. Ausbeute 80%.
¹H NMR (300 MHz, DMSO): δ = 0.80-0.90 ppm (2 d, 6H, CH₃ 8 und 9), 1.45-1.80 ppm (m, 3H, CH 7 und CH₂ 6), 4.20-4.30 ppm (CH₂ 2 in der Endgruppe), 4.30-4.50 ppm (CH 5), 4.50-4.73 ppm (AB-System, ^{AB}J = 14.6 Hz, 2H, CH₂ 2, isot.), 5.49-5.55 ppm (t, ³J = 5.8 Hz 1H, OH 1), 8.30-8.40 ppm (d, ³J = 7.7 Hz 1H, NH 4); Starter: δ = 3.80-3.90 ppm (d, ³J = 5.7 Hz, 4H, CH₂ 11 and 12).
¹³C NMR (75.41 MHz, DMSO): δ = 21.1 ppm (CH₃ 8 oder 9), 22.8 ppm (CH₃ 8 oder 9), 24.1 ppm (CH 7), 40.4 ppm (CH₂ 6), 49.9 ppm (CH 5), 62.1 (CH₂ 2), 166.6 ppm (COO 10), 171.7 ppm (CONH 3, syndiot.), 171.8 ppm (CONH 3, isot.), 172.3 (CONH 3 Endgruppe); Starter: δ = 61.2 ppm (CH₂ 11 und 12).
Mₙ = 6.300 g/mol (¹H NMR), 5.700 g/mol (OH Zahl-Bestimmung)

### Synthese von Poly(3S-sec-butyl-morpholin-2,5-dion)diol (PBMD)

Die Herstellung erfolgte in zur Herstellung von PIBMD analoger Verfahrensführung, jedoch mit den Ausgangsstoffen 3S-sec-Butyl-morpholin-2,5-dion und 1,8-Octandiol. 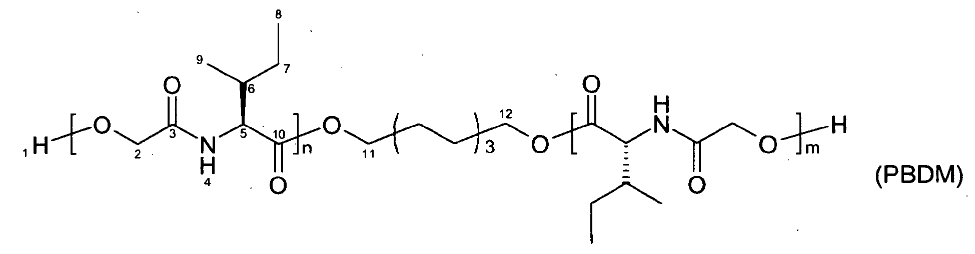 ¹H NMR (300 MHz, CDCl₃): δ = 0.90-1.09 ppm (2 d, 6H, CH₃ 8 und 9), 1.21-1.75 ppm (m, 2H, CH₂ 7), 1.96-2.04 ppm (m, 1H, CH 6), 4.10-4.20 ppm (CH₂ 2 Endgruppe), 4.24-4.30 ppm (m, 1H, CH 5), 4.43-4.90 ppm (AB-System, ^{AB}J = 14.6 Hz, 2H, CH₂ 2, isot.), 7.50-7.70 ppm (1H, NH 4); Starter: δ = 4.05-4.10 ppm (4H, CH₂ 11 und 12).

### Synthese von Poly(3-methyl-morpholin-2,5-dion)diol (PMMD)

Die Herstellung erfolgte in zur Herstellung von PIBMD analoger Verfahrensführung, jedoch mit den Ausgangsstoffen 3-Methyl-morpholin-2,5-dion und 1,8-Octandiol. 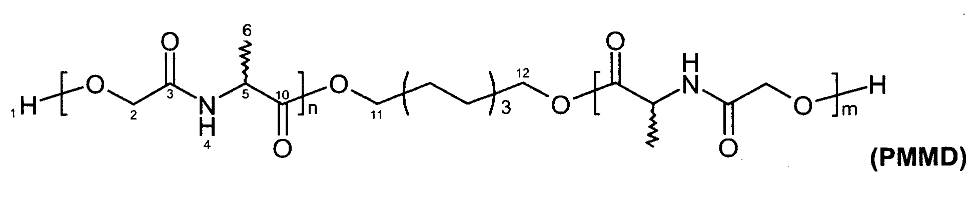 ¹H NMR (300 MHz, DMSO): δ = 1.2-1.4 ppm (d, 3H,CH₃6), 4.3-4.4 ppm (m, 1H, CH 5), 4.5-4.7 ppm (m, 2H, CH₂ 2), 8.3-8.5 ppm (2 d, 1H, NH 4); Starter: δ = 3.8-3.9 ppm (m, 4H, CH₂ 11 und 12).

Der Tabelle 1 sind ausgewählte Eigenschaften der Polymere PIBMD, PBMD und PMMD zu entnehmen.

**Tab. 1**

| Polymer | Ausbeute in % | M_{OH}¹⁾ | M_{NMR}²⁾ | M_{n,GPC}³⁾ | D ⁴⁾ | Tₘ⁵⁾ in °C | ΔH ⁵⁾ in J/g | T_{g}⁵⁾ in °C |
|---|---|---|---|---|---|---|---|---|
| PIBMD | 80 | 5700 | 6300 | 3300 | 2.1 9 | 170 | 20.3 | 43 |
| PBMD | 80 | 2730 | 1900 | 1800 | 2.7 1 | 80 | 24.3 | 50 |
| PMMD | 88 | 5600 | 3100 | 10500 | 1.1 6 | 114 | 24.5 | 62 |

| | | | | | | | | |
|---|---|---|---|---|---|---|---|---|
| 1) Molekulargewicht durch Bestimmung der OH-Zahl. 2) Molekulargewicht anhand ¹H NMR-Spektrum. 3) Molekulargewicht GPC. 4) Molekulargewichtsverteilung GPC. 5) Dynamische Differenz-Kalorimetrie (DSC). | | | | | | | | |

### Synthese des Multiblockcopolymers PCL/PIBMD

Ein Gemisch aus 24.0 g (12 mmol) PCL (Poly(ε-Caprolacton); Handelsname CAPA2304 von Solvay Caprolactones, UK; mittleres Molekulargewicht M_{w} 3000 g/mol), 22.5 g (4 mmol) PIBMD, 16 mmol TMDI, 43 µL Dibutylzinndilaurat (ca. 0.1 Gew.%) und 110 g N-Methylpyrrolidon wurde in einem Zweihalsrundkolben unter Stickstoff und kontinuierlichen Rühren mittels eines Magnetrührers zugesetzt. Es wurde auf 80°C erhitzt und nach 24 h wurde das Reaktionsgemisch mittels IR-Spektroskopie und Gelpermeationschromatographie (GPC) analysiert. Nachdem die NCO-Bande im IR bei 2270 cm⁻¹ verschwunden war, wurden 100 µL TMDI zugesetzt und es wurde für weitere 24 h gerührt. Anschließend wurde das Reaktionsgemisch mit 200 mL 1,2-Dichloroethan und mit dem 10-fachen Überschuss an Diethylether ausgefällt. Das gefällte Multiblockcopolymer wurde durch Filtration gesammelt und unter Vakuum bei RT für 24 h getrocknet. Ausbeute 90%.
¹H NMR (300 MHz, DMSO): PIBMD Block: δ = 0.80-0.90 ppm (2 d, 6H, CH₃ 8 und 9), 1.45-1.80 ppm (m, 3H, CH und CH₂), 4.30-4.50 ppm (CH), 4.50-4.73 ppm (AB-System, ^{AB}J = 14.6 Hz, 2H, CH₂, isot.), 8.30-8.40 ppm (d, ³J = 7.7 Hz 1H, NH); Starter: δ = 3.82-3.90 ppm (d, ³J = 5.7 Hz, 4H, CH₂); PCL Block: δ = 1.23-1.37 ppm (m, 2H, CH₂), 1.46-1.71 ppm (m, 4H, CH₂, überlappend mit PIBMD Block), 2.23-2.30 ppm (t, ³J = 7.3 Hz 2H, CH₂), 3.94-4.01 ppm (t, ³J = 6.6 Hz 2H, CH₂); Starter: δ = 3.57-3.62 ppm (m, 4H, CH₂) und 4.08-4.13 ppm (m, CH₂); TMDI: δ = 0.76-0.93 ppm (m, CH₃, überlappend mit PIBMD Block), 1.05-1.19 ppm (m, CH₂ und CH), 2.68-3.02 ppm (m, CH₂).

### Synthese des Multiblockcopolymers PCL/PMMD

Die Herstellung erfolgte in zur Herstellung von PCL/PIBMD analoger Verfahrensführung.
¹H NMR (300 MHz, CDCl₃): PMMD Block: δ = 1.3-1.4 ppm (CH₃), 4.3-4.4 ppm (s, CH), 4.5-4.7 ppm (CH₂), 7.6-8.0 ppm (NH); Starter: δ = 3.6 ppm (CH₂). PCL Block: δ = 1.4-1.5 ppm (m, CH₂ überlappend mit PMMD Block), 1.5-1.7 ppm (m, CH₂), 2.2-2.40 ppm (2H, CH₂), 4.0-4.1 ppm (2H, CH₂); Starter: δ = 3.6-3.7 ppm (m, 4H, CH₂) and 4.2 ppm (m, CH₂). TMDI: δ = 0.80-0.90 ppm (m, CH₃), 0.9-1.0 ppm (m, CH₂ und CH), 2.8-3.2 ppm (m, CH₂).

Der Tabelle 2 sind ausgewählte Eigenschaften der Multiblockcopolymere PCL/PIBMD und PCL/PMMD zu entnehmen.

**Tab. 2**

| Multiblock-copölymer | Poly(depsi-peptid) Gew.% | M_{n,GPC}¹⁾ | D ²⁾ | Tₘ₁³⁾ [C°] | ΔH₁⁴⁾ [J/g] | Tₘ₂⁵⁾ [C°] | ΔH₂⁶⁾ [J/g] | T_{g}⁷⁾ [C°] |
|---|---|---|---|---|---|---|---|---|
| PCL/ PIBMD | 50 | 62000 | 1.6 5 | 37.9 | 53.2 | 170 | 47.8 | -60 |
| PCL/ PMMD | 50 | 23000 | 1.4 7 | 44 | 59.4 | 80 | 72 | 28 und 51⁸⁾ |

| | | | | | | | | |
|---|---|---|---|---|---|---|---|---|
| 1) Molekulargewicht GPC. 2) Molekulargewichtsverteilung GPC 3) erster Peak im DSC Diagramm 4) Enthalpie des ersten Peaks im DSC Diagramm 5) zweiter Peak im DSC Diagramm 6) Enthalpie des zweiten Peaks im DSC Diagramm 7) Glasübergangstemperatur aus DSC, erster Lauf. 8) Glasübergangstemperatur aus DSC, zweiter Lauf. | | | | | | | | |

### Herstellung eines Probefilms

Ein Film mit einer Dicke von 400 µm wurde aus dem Multiblockcopolymer PCUPIBMD durch Kompressionsschmelzen bei 180°C und 90 bar hergestellt. Das DSC des PCUPIBMD-Films zeigte, dass die Enthalpie der PIBMD-Blöcke sehr niedrig war. Die PIBMD-Blöcke müssen eine hohe Kristallinität aufweisen, um die permanente Form des Films zu fixieren. Um die Kristallinität der PIBMD-Blöcke zu erhöhen, wurde der Film bei 100 °C für 30 min und bei 80 °C für 24 Stunden getempert und dann langsam auf Raumtemperatur abgekühlt.

Das aus Poly(ε-caprolacton)-Blöcken (PCL-Blöcken) und PIBMD-Blöcken aufgebaute Multiblockcopolymer PCUPIBMD wurde unter Einsatz von 2,2,4- und 2,4,4-Trimethylhexamethylendiisocyanat (TMDI) als Kupplungsreagenz synthetisiert.

Die von den PCL-Blöcken bestimmte Phase mit einer Schmelztemperatur von etwa 37°C fungiert als Schaltsegmente während die von den PIBMD-Blöcken bestimmte kristalline Phase mit der höheren Schmelztemperatur das Hartsegment darstellt. Die Topographie und das Phasenverhalten des Multiblockcopolymers wurden mittels Rasterkraftmikroskopie (AFM) anhand eines auf einem Silikonsubstrat aufgebrachten Polymerfilms untersucht. Die Oberflächentopographien der Proben wurden bei Raumtemperatur untersucht, um die Oberflächenmorphologie oberhalb der PCL-Schmelztemperatur zu erfassen. Anschließend wurden die Proben wieder auf Raumtemperatur abgekühlt.

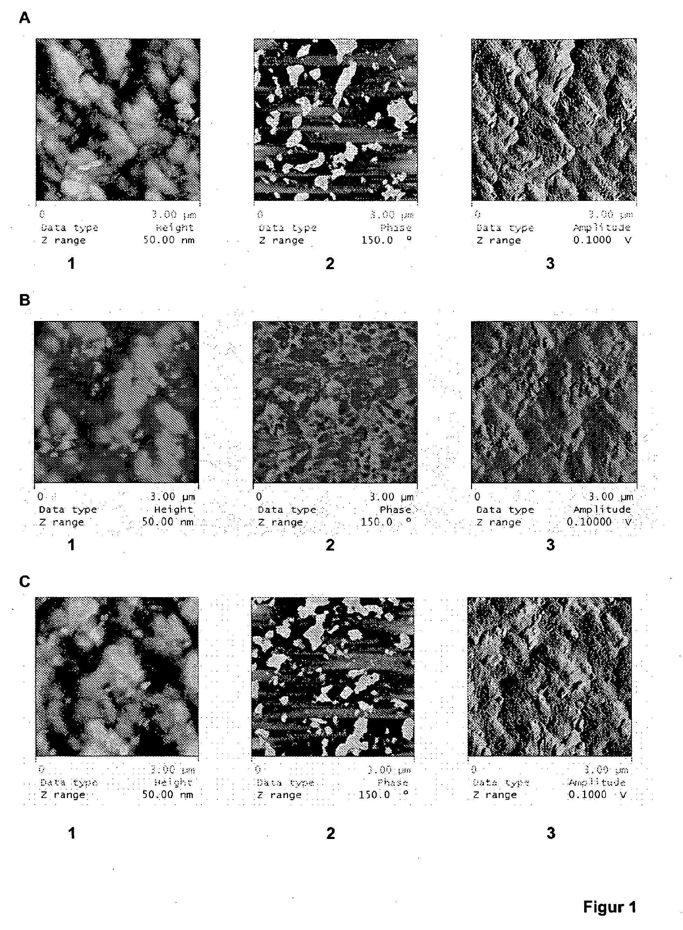

Der **Figur 1** sind AFM-Abbildungen der PCL/PIBMD-Oberfläche bei verschiedenen Temperaturen, nämlich A = Raumtemperatur, B = 60°C, C = Raumtemperatur nach dem Abkühlen von 60°C zu entnehmen. Punkt 1 ist dabei eine Darstellung der Oberflächentopographie, Punkt 2 eine Phasendarstellung und Punkt 3 eine Amplitudendarstellung. In der Phasendarstellung 2 entspricht der dunkle Bereich dem Hartsegment und der helle Bereich dem Schaltsegment.

Die PCL-Domänen hatten eine Ausdehnung von bis zu 400 nm, wohingegen PIBMD-Blöcke in kontinuierlicher Phase vorlagen. Der Vergleich der Topographie und der Phasendarstellung zeigt, dass die Phasendomäne nicht die Topographie des Films beeinflusst. Nach dem Abkühlen auf Raumtemperatur rekristallisierte die PCL-Phase und die AFM-Aufnahme glich den Aufnahmen vor dem Erhitzen. Demnach zeigt das Multiblockcopolymer PCL/PIBMD eine Mikrophasen-Separation zwischen der PCL- und PIBMD-Phase, die zur Bildung einer Art Nano-Komposit führt zwischen den das Schaltsegment bestimmenden PCL-Blöcken und den das Hartsegment bestimmenden PIBMD-Blöcken.

### Thermische Eigenschaften von PCL-PIBMD

Eine thermische Analyse mittels DSC-Messung der Multiblockcopolymere bestätigte, dass diese semikristallin sind. PCL-diol 3K weist einen doppelten Schmelzpunkt bei 48°C und 50°C (ΔH = 60,5 J/g) und einen Glasübergang bei etwa -60 °C auf. PIBMD 5K hat eine Schmelztemperatur von etwa 170°C (ΔH = 20,3 J/g) und einen Glasübergang bei 43°C. Die beiden Schmelztemperaturen im Multiblockcopolymer lagen bei 170°C und 34°C für die PIBMD-Blöcke beziehungsweise PCL-Blöcke. Die kristalline PIBMD-Phase verhindert eine Kristallisation der PCL-Blöcke. Im zweiten Aufheizvorgang rekristallisierte PIBMD bei etwa 101°C und zeigte bei 170°C (39,4 J/g) einen Schmelzübergang, während die PCL-Phase eine Schmelztemperatur von 37°C aufwies (3,0 J/g).

### Mechanische Eigenschaften des Multiblockcopolymers PCL/PIBMD

Die mechanischen Eigenschaften der PCL/PIBMD Multiblockcopolymere wurden mittels Zug-Dehnungs-Versuchen oberhalb und unterhalb von Tₘ der PCL-Blöcke untersucht. Ergebnisse dieser Versuche sind in Tabelle 3 zusammengetragen.

**Tab. 3**

| E^{[a]} [MPa] | σ_{b}¹⁾ [MPa] | ε_{b}¹⁾ [%] | E²⁾ [MPa] | σ_{b}²⁾ [MPa] | ε_{b}²⁾ [%] | εₘ [%] | R*_{f}*(1) [%] | Rᵣ(1) [%] | *R̅*^{*f,*2-5} [%] | *R̅*^{r,2-5} [%] |
|---|---|---|---|---|---|---|---|---|---|---|
| 72±12 | 14.5±2.1 | 420±80 | 30.4±9.0 | 2.8±0.1 | 70±11 | 50 | 97.8 | 97.1 | 96.3 | 98.8 |

| | | | | | | | | | | |
|---|---|---|---|---|---|---|---|---|---|---|
| 1) Bestimmung bei 25°C. 2) Bestimmung bei 75°C. | | | | | | | | | | |

### Formgedächtniseigenschaften von PCUPIBMD

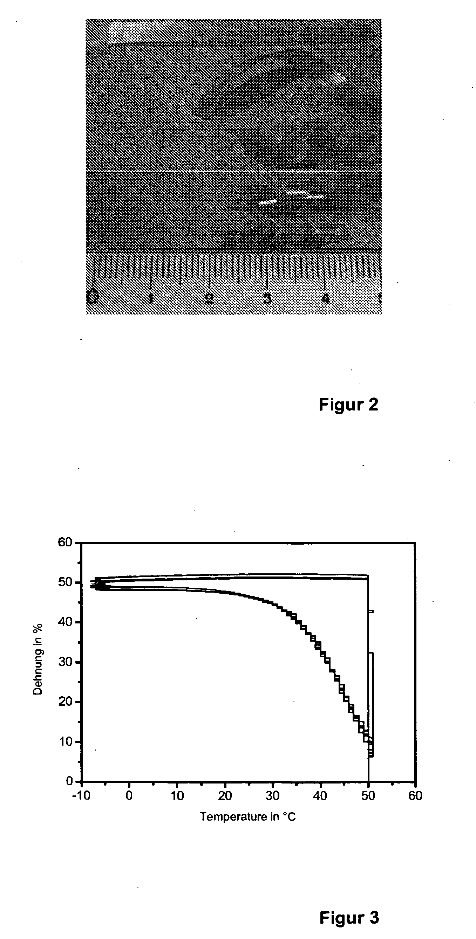

Ein in seiner permanenten Form als helixartig verdrilltes Band vorliegendes PCL/PIBMD Multiblockcopolymer wurde ausgehend von seiner permanenten Form bei hoher Temperatur (T = 120°C) in die temporäre Form (flacher Polymerstreifen) überführt. Die deformierte Form wurde durch Abkühlen auf Raumtemperatur fixiert. Zur Wiederherstellung der permanenten Form wurde die Probe über die Schalttemperatur Tₜᵣₐₙₛ (auf circa 60°C) erhitzt und die ursprüngliche permanente Form wieder hergestellt. Der makroskopische Formgedächtniseffekt von PCL-PIBMD ist der **Figur 2** zu entnehmen.

Die Formgedächtniseigenschaften des Multiblockcopolymers PCL-PIBMD wurden mittels zyklischer thermomechanischer Untersuchungen quantifiziert, wobei eine maximale Dehnung von εₘ = 50 % angewendet wurde. Die Messresultate von fünf aufeinander folgenden Thermozyklen sind **Figur 3** zu entnehmen. Die Überlagerung der Kurven (N=2-5) zeigt an, dass die Formgedächtniseigenschaften nach Durchlaufen des ersten Zyklus (N=1) konstante Werte annehmen, so dass signifikante Relaxationseffekte oder das Auftreten von irreversiblen Effektenwährend der thermomechanischen Untersuchung ausgeschlossen werden können.

## Patentansprüche

1. Multiblockcopolymer mit Formgedächtniseigenschaften, enthaltend
(i) ein Poly(depsipeptid)-Segment mit einem mittleren Molekulargewicht M_{w} im Bereich von 1.000 bis 20.000 g/mol; sowie
(ii) ein Poly(ε-caprolacton)-Segment mit einem mittleren Molekulargewicht M_{w} im Bereich von 1.000 bis 10.000 g/mol.

2. Multiblockcopolymer nach Anspruch 1, mit einem Poly(depsipeptid)-Segment der Formel (1): 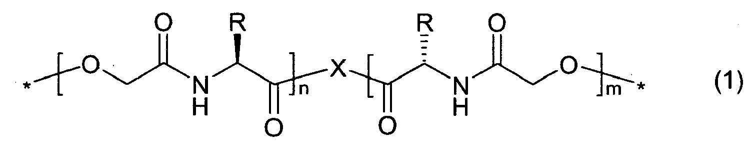 wobei X eine Brücke ausgewählt aus der Gruppe: 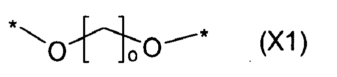 und 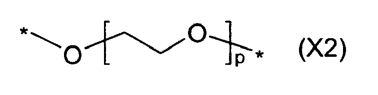 ist, mit o = 2 - 20 und p = 1 - 10;
R für eine Gruppe ausgewählt aus H oder einem verzeigten oder unverzweigten C₁-C₁₀-Alkylrest steht; und
n und m so vorgegeben sind, dass das Poly(depsipeptid)-Segment ein mittleres Molekulargewicht M_{w} im Bereich von 1.000 bis 20.000 g/mol aufweist.

3. Multiblockcopolymer nach Anspruch 2, bei dem X für 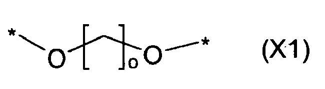 steht und o = 8 ist.

4. Multiblockcopolymer nach Anspruch 2, bei dem X für 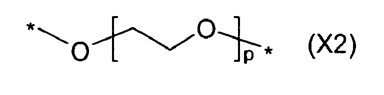 steht und p = 1 ist.

5. Multiblockcopolymer nach einem der Ansprüche 2 bis 4, bei dem R für H, Methyl, 1-Methylethyl, 2-Methylpropyl oder 1-Methylpropyl steht.

6. Multiblockcopolymer nach einem der vorhergehenden Ansprüche, mit einem Poly(ε-caprolacton)-Segment der Formel (2) 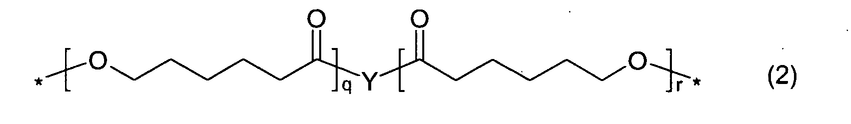 ist, wobei Y für 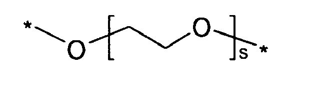 steht, mit s = 1 - 10; und
q und r so vorgegeben sind, dass das Poly(ε-caprolacton)-Segment ein mittleres Molekulargewicht M_{w} im Bereich von 1.000 bis 10.000 g/mol aufweist.

7. Multiblockcopolymer nach Anspruch 6, bei dem s = 2 ist.

8. Multiblockcopolymer nach einem der Ansprüche 2 bis 7, bei dem die Poly(depsipeptid)-Segmente und Poly(ε-caprolacton)-Segmente im Multiblockcopolymer über Brücken der Formeln (3a) und/oder (3b) 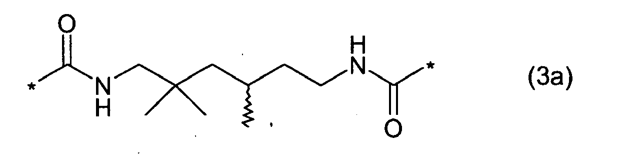 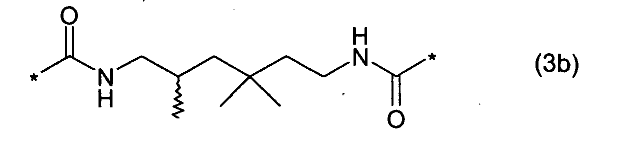 gekuppelt sind.

9. Multiblockcopolymer nach einem der Ansprüche 2 bis 8, bei dem ein Gewichtsverhältnis der Poly(depsipeptid)-Segmente zu den Poly(ε-caprolacton)-Segmenten im Bereich von 1:1 bis 1:10 liegt.

10. Multiblockcopolymer nach einem der vorhergehenden Ansprüche, mit einem mittleren Molekulargewicht M_{w} des Multiblockcopolymers im Bereich von 10.000 bis 100.000 g/mol.

11. Poly(depsipeptid) der Formel (4): 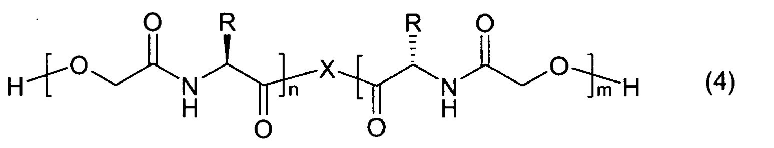 wobei X eine Brücke ausgewählt aus der Gruppe: 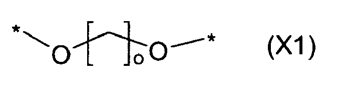 und 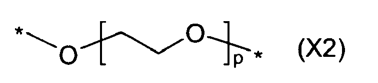 ist, mit o = 2 - 20 und p = 1 - 10;
R für eine Gruppe ausgewählt aus H oder einem verzeigten oder unverzweigten C₁-C₁₀-Alkylrest steht; und
n und m so vorgegeben sind, dass das Poly(depsipeptid) ein mittleres Molekulargewicht M_{w} im Bereich von 1.000 bis 20.000 g/mol aufweist.

12. Verwendung eines Multiblockcopolymers nach einem der Ansprüche 1 bis 10 als Implantatwerkstoff, als Polymermatrix für die kontrollierte Wirkstofffreisetzung (Wirkstoffdepots und Coatings zur Wirkstoffverkapselung) und als Material zur Herstellung von Gerüst- und Leitstrukturen (Polymer Scaffolds und alloplastische Leitschienen) für das Tissue Engineering.

## Claims

1. Multiblock copolymer with shape memory properties, containing
(i) a poly(depsipeptide) segment with an average molecular weight M_{w} in the range from 1000 to 20 000 g/mol; and
(ii) a poly(ε-caprolactone) segment with an average molecular weight M_{w} in the range from 1000 to 10 000 g/mol.

2. Multiblock copolymer according to Claim 1 with a poly(depsipeptide) segment of formula (1): 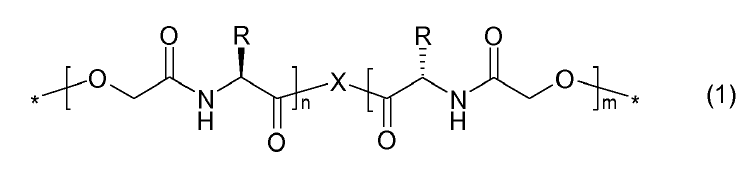 where X is a bridge selected from the group: 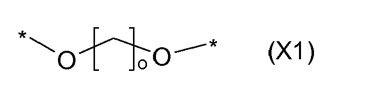 and 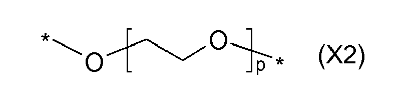 where o = 2-20 and p = 1-10;
R represents a group selected from H or a branched or unbranched C₁-C₁₀ alkyl radical; and
n and m are predetermined such that the poly(depsipeptide) segment has an average molecular weight M_{w} in the range from 1000 to 20 000 g/mol.

3. Multiblock copolymer according to Claim 2, wherein X represents 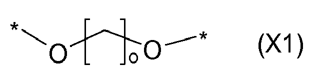 and o is = 8.

4. Multiblock copolymer according to Claim 2, wherein X represents 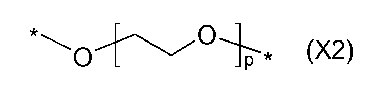 and p is = 1.

5. Multiblock copolymer according to any one of Claims 2 to 4, wherein R represents H, methyl, 1-methylethyl, 2-methylpropyl or 1-methylpropyl.

6. Multiblock copolymer according to any preceding claim with a poly(ε-caprolactone) segment of formula (2) 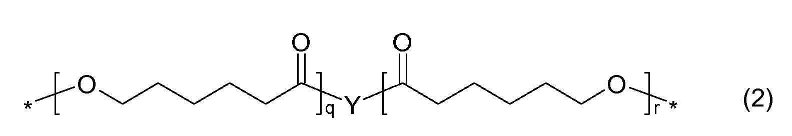 where Y represents 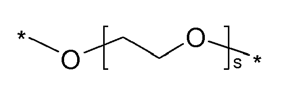 where s = 1-10; and
q and r are predetermined such that the poly(ε-caprolactone) segment has an average molecular weight M_{w} in the range from 1000 to 10 000 g/mol.

7. Multiblock copolymer according to Claim 6, wherein s is = 2.

8. Multiblock copolymer according to any one of Claims 2 to 7, wherein the poly(depsipeptide) segments and poly(ε-caprolactone) segments in the multiblock copolymer are coupled via bridges of formulae (3a) and/or (3b)

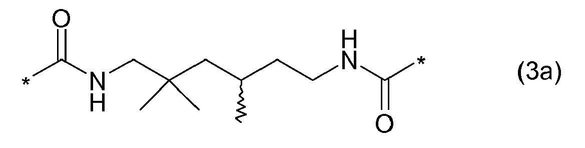

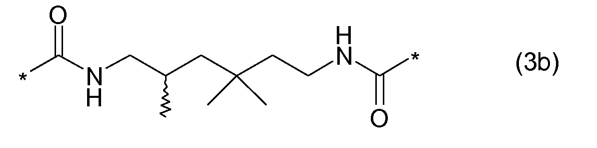

9. Multiblock copolymer according to any one of Claims 2 to 8, wherein a weight ratio of the poly(depsipeptide) segments to the poly(ε-caprolactone) segments is in the range from 1:1 to 1:10.

10. Multiblock copolymer according to any preceding claim, with an average molecular weight M_{w} of the multiblock copolymer in the range from 10 000 to 100 000 g/mol.

11. Poly(depsipeptide) of formula (4): 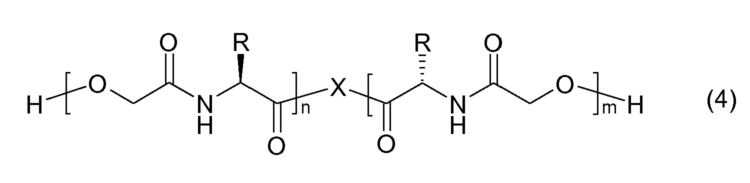 where X is a bridge selected from the group: 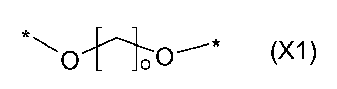 and 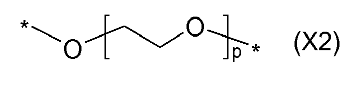 where o = 2-20 and p = 1-10;
R represents a group selected from H or a branched or unbranched C₁-C₁₀ alkyl radical; and
n and m are predetermined such that the poly(depsipeptide) has an average molecular weight M_{w} in the range from 1000 to 20 000 g/mol.

12. Use of a multiblock copolymer according to any one of Claims 1 to 10 as an implant material of construction, as a polymer matrix for the controlled release of an active ingredient (active-ingredient depots and coatings for active-ingredient encapsulation) and as a material in the manufacture of scaffolds (polymer scaffolds and alloplastic scaffolds) for tissue engineering.

## Revendications

1. Copolymère à blocs multiples avec des propriétés de mémoire de forme, contenant
(i) un segment poly(depsipeptide) présentant un poids moléculaire moyen M_{w} dans la zone de 1000 à 20 000 g/mole ; ainsi que
(ii) un segment poly(ε-caprolactone) présentant un poids moléculaire moyen M_{w} dans la plage de 1000 à 10 000 g/mole.

2. Copolymère à blocs multiples selon la revendication 1, présentant un segment poly(depsipeptide) de formule (1) : 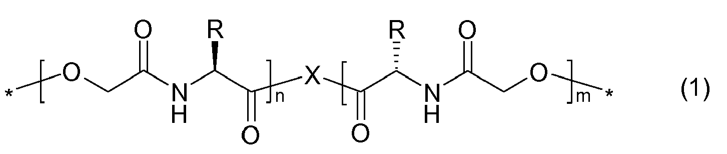 où X représente un pont choisi dans le groupe : 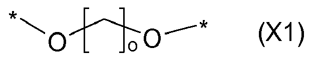 et 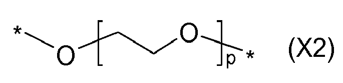 avec o = 2 - 20 et p = 1 - 10 ;
R représente un groupe choisi parmi H ou un radical C₁-C₁₀-alkyle ramifié ou non ramifié ; et
n et m sont fixés de manière telle que le segment poly(depsipeptide) présente un poids moléculaire moyen M_{w} dans la plage de 1000 à 20 000 g/mole.

3. Copolymère à blocs multiples selon la revendication 2, dans lequel X représente 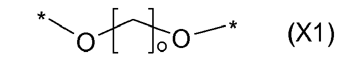 et o = 8.

4. Copolymère à blocs multiples selon la revendication 2, dans lequel X représente 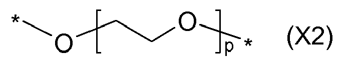 et p = 1.

5. Copolymère à blocs multiples selon l'une quelconque des revendications 2 à 4, dans lequel R représente H, méthyle, 1-méthyléthyle, 2-méthylpropyle ou 1-méthylpropyle.

6. Copolymère à blocs multiples selon l'une quelconque des revendications précédentes, présentant un segment poly(ε-caprolactone)de formule (2) : 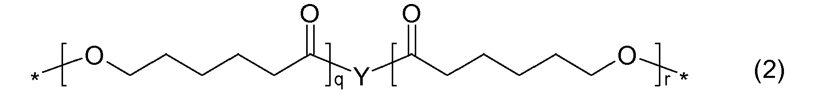 où Y représente 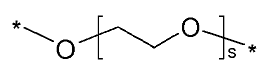 avec s = 1 - 10 ; et
q et r sont fixés de manière telle que le segment poly(ε-caprolactone) présente un poids moléculaire moyen M_{w} dans la plage de 1000 à 10 000 g/mole.

7. Copolymère à blocs multiples selon la revendication 6, dans lequel s = 2.

8. Copolymère à blocs multiples selon l'une quelconque des revendications 2 à 7, dans lequel les segments poly(depsipeptide) et les segments poly(ε-caprolactone) dans le copolymère à blocs multiples sont couplés via des ponts des formules (3a) et/ou (3b)

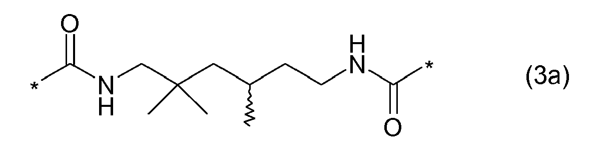

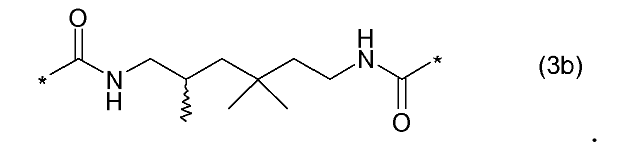

9. Copolymère à blocs multiples selon l'une quelconque des revendications 2 à 8, dans lequel un rapport pondéral des segments poly(depsipeptide) aux segments poly(ε-caprolactone) se situe dans la plage de 1:1 à 1:10.

10. Copolymère à blocs multiples selon l'une quelconque des revendications précédentes, présentant un poids moléculaire moyen M_{w} du copolymère à blocs multiples dans la plage de 10 000 à 100 000 g/mole.

11. Poly(depsipeptide) de formule (4) : 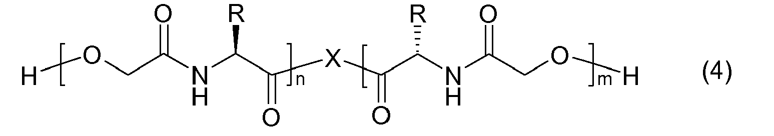 où X représente un pont choisi dans le groupe : 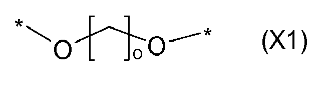 et 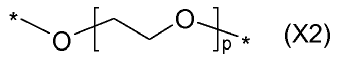 avec o = 2 - 20 et p = 1 - 10 ;
R représente un groupe choisi parmi H ou un radical C₁-C₁₀-alkyle ramifié ou non ramifié ; et
n et m sont fixés de manière telle que le poly(depsipeptide) présente un poids moléculaire moyen M_{w} dans la plage de 1000 à 20 000 g/mole.

12. Utilisation d'un copolymère à blocs multiples selon l'une quelconque des revendications 1 à 10 comme matériau pour implant, comme matrice polymère pour la libération contrôlée de substance(s) active(s) (substance(s) active(s) à effet de retard et revêtement pour l'enrobage de substance(s) active(s)) et comme matériau pour la production de structures de treillis et de guidage (Polymer Scaffolds et structures de guidage alloplastiques) pour l'ingénierie tissulaire.
